(19) Europäisches Patentamt
European Patent Office
Office européen des brevets

(11) Publication number: **0 005 867**
**B2**

(12) # NEW EUROPEAN PATENT SPECIFICATION

(45) Date of publication of the new patent specification: **09.07.86**

(51) Int. Cl.⁴: **G 01 N 33/50**, C 12 Q 1/40

(21) Application number: **79101791.6**

(22) Date of filing: **06.06.79**

(54) **An alpha-amylase assay, a reagent composition and a reagent system therefor.**

(30) Priority: **06.06.78 US 912986**

(43) Date of publication of application:
**12.12.79 Bulletin 79/25**

(45) Publication of the grant of the patent:
**27.10.82 Bulletin 82/43**

(45) Mention of the opposition decision:
**09.07.86 Bulletin 86/28**

(84) Designated contracting states:
**DE FR GB IT**

(56) References cited:
**DE-A- 2 705 859**
**DE-A- 2 729 636**
**FR-A- 2 402 872**
**US-A- 3 879 263**
**Clinical Chemistry, vol. 24, no. 6, (1978), page 1000**

(73) Proprietor: **FLOW GENERAL, INC.**
**7655 Old Springhouse Road**
**Mclean Virginia 22102 (US)**

(72) Inventor: **Nix, Paul Thomas**
**62 Forest Drive**
**Jackson N.J. 08627 (US)**
Inventor: **Goldfarb, Rebecca Dickstein**
**224 Alexander Avenue**
**Howell N.J. 07731 (US)**
Inventor: **Morgenstern, Stanley Walter**
**3805 Route 33**
**Neptune N.J. 07753 (US)**
Inventor: **Stong, Linda Jean**
**6 Blue Spruce Court R.D. 1**
**Hightstown N.J. 08520 (US)**
Inventor: **Sulick, Lorraine Ellen**
**14 Winding Way**
**Short Hills N.J. 07078 (US)**
Inventor: **Trivedi, Ramesh Chandulal**
**110 W.Main Street**
**Freehold N.J. 07728 (US)**

(74) Representative: **Vossius Vossius Tauchner Heunemann Rauh**
**Siebertstrasse 4 P.O. Box 86 07 67**
**D-8000 München 86 (DE)**

**EP 0 005 867 B2**

**Description**

This invention relates to reagents and methods for determining alpha-amylase concentration in a body fluid, such as plasma, serum, urine and saliva. It relates more especially to the detection and measurement of alpha-amylase using coupled enzymatic reactions and to the substrate for alpha-amylase that is used therein.

Background

Alpha-amylase determinations are being resorted to with increasing frequency in clinical laboratories as an aid in diagnosing pancreatic and other diseases which alter normal concentration of alpha-amylase in a body fluid. Alpha-amylase is the common traditional name for the enzyme more precisely designated as alpha-1,4-glucan 4-glucanohydrolase.

Alpha-amylase plays a vital physiological role in enabling the digestion of starch and other polysaccharides by virtue of its specific enzymatic activity in the hydrolysis of alpha-1,4 linkages in the polysaccharide molecule, with the formation of maltose and various other oligosaccharides as ultimate products of the enzymatic reaction. Detection of abnormalities in the alpha-amylase content of body fluid provides an important aid in clinical diagnosis.

A number of analytical methods for the determination of alpha-amylase have been developed based upon the ability of alpha-amylase to hydrolyze starch. In all of these methods, starch or a starch derivative is digested with the sample containing alpha-amylase to be analyzed. Then the extent of the alpha-amylase induced degradation is measured by a variety of procedures. For example, the decrease in the amount of the starch substrate has been directly measured by an amyloclastic method, in which the decreased starch content is determined by adding iodine to the sample, and measuring the intensity of the blue colored starch-iodine complex which is formed. Alternatively, the decrease in starch content has been measured by the decrease in turbidity of an aqueous starch suspension following alpha-amylase digestion. Another chemical method for determining alpha-amylase activity involves use of a modified starch as the substrate, wherein the starch has a chromophore covalently linked to it. On digestion with alpha-amylase, small, water soluble colored fragments are formed, and the intensity of the color in the solution is measured photometrically.

Still another chemical analytical procedure is based upon reduction-oxidation analysis for reducing ends which are formed by the reaction of alpha-amylase with the starch substrate.

None of these chemical methods has proven entirely satisfactory for clinical use. All are time consuming, and require standard curves, while most also require the use of blanks, and long incubation times. Other problems include variability depending upon the source of the starch, sensitivities due to differences in laboratory technique and the need for specialized equipment.

Still another analyses are based upon enzymatic detection methods and, to date, all prior commercially available enzymatic alpha-amylase kits have been based upon measurements of the rate at which maltose is formed by the action of alpha-amylase on starch or an oligosaccharide substrate, such as maltotetraose or maltopentose. In one such analysis, maltose in converted to glucose by the enzyme alpha-glucosidase, and the level of glucose is in turn determined by conversion to glucose-6-phosphate by hexokinase, and conversion of glucose-6-phosphate to 6-phosphogluconate by reaction with beta-nicotinamide-adenine-dinucleotide (NAD) in the presence of glucose-6-phosphate dehydrogenase, as represented by the following sequence :

$$\text{Maltose} \xrightarrow{\text{alpha-glucosidase}} \text{glucose.} \qquad (1)$$

$$\text{Glucose} + \text{ATP} \xrightarrow{\text{hexokinase}} \text{glucose-6-phosphate} + \text{ATP.} \qquad (2)$$

$$\text{Glucose-6-phosphate} + \text{NAD} \xrightarrow[\text{dehydrogenase}]{\text{glucose-6-phosphate}} \text{6-phosphogluconate} + \text{NADH.} \qquad (3)$$

In this reaction system the conventional abbreviation NAD is used and is used elsewhere herein and in the claims, in reference to the coenzyme beta-nicotinamide-adenine-dinucleotide and NADH is used in reference to the same coenzyme in its reduced form. It also is to be understood that the NAD may be replaced by or used in admixture with NADP, namely, beta-nicotinamide-adenine-dinucleotide phosphate which in its reduced form is indicated as NADPH.

Still other procedures detect the formation of maltose via the enzyme maltose phosphorylase, as is illustrated by the following sequence :

$$\text{Maltose} + \text{phosphate} \xrightarrow{\text{maltose phosphorylase}} \text{glucose} + \text{beta-glucose-1-phosphate.} \quad (1)$$

$$\text{Beta-glucose-1-phosphate} \xrightarrow{\text{beta-phosphoglucomutose}} \text{glucose-6-phosphate.} \quad (2)$$

$$\text{Glucose-6-phosphate} + \text{NAD} \xrightarrow[\text{dehydrogenase}]{\text{glucose-6-phosphate}} \text{6-phosphogluconate} + \text{NADH.} \quad (3)$$

Typically, the increase in absorbence at 340 nm due to increased levels of NADH is measured photometrically. These methods still are not as accurate or as sensitive as may be desired.

Still more recently, it has been proposed to use as the substrate for the alpha-amylase a starch which has been subjected to partial oxidation, with resulting damaging random effect on alpha-1,4 linked glucoses of the starch molecule to form a so-called « blocked » starch. In such a « blocked starch », the reactivity of the damaged alpha-1,4 linkages is blocked to sufficient extent to inhibit the activity of an exocarbohydrase on the starch molecule, while at the same time leaving residual alpha-1,4 linkages that are subject to alpha-amylase attack with attendant liberation of free non-reducing chain terminals that can be attacked by an exocarbohydrase, such as phosphorylase, which serves as a coupling enzyme in the following enzymatic reaction system :

$$\text{« Blocked » starch} \xrightarrow{\text{alpha-amylase}} \text{« unblocked starch ».} \quad (1)$$

$$\text{« Unblocked starch »} + \text{inorganic phosphate} \xrightarrow{\text{phosphorylase}} \text{glucose-1-phosphate} + \text{degraded starch} \quad (2)$$

$$\text{Glucose-1-phosphate} \xrightarrow{\text{phosphoglucomutase}} \text{glucose-6-phosphate.} \quad (3)$$

$$\text{Glucose-6-phosphate} + \text{NAD} \xrightarrow[\text{dehydrogenase}]{\text{glucose-6-phosphate}} \text{NADH} + \text{6-phosphogluconate.} \quad (4)$$

The use of « blocked » starch in providing a substrate comprised in a coupled enzymatic reaction system is attended with inherent disadvantages in that, in producing the blocked starch substrate, the oxidation of the starch is only partial and damages alpha-1,4 linkages randomly throughout the starch molecule. As a result, difficulties are encountered as regards controlling the uniformity of the substrate and its reactivity with alpha-amylase and, as a corollary, correctly indicating the rate of change in absorbency occasioned by alpha-amylase in a body fluid. Moreover, because of the reduced overall activity occasioned by the partial oxidation, the amount of the « blocked » starch required for accomplishing a given change in absorbence is undesirably large and substantially interferes with the sensitivity of the test determination (DE-A-2 729 636 and C. Y. Huang, Clinical Chemistry, *22,* 1164 (Article 023) 1976).

It is an object of this invention to provide for use, in determining the concentration of alpha-amylase in a body fluid, an improved enzymatic reagent system that is responsive to the presence of alpha-amylase in a body fluid. It also is an object of this invention to provide a new reagent composition for use in said system and an improved substrate for alpha-amylase for use in the reagent system.

Further objects of the invention relate to improvements whereby the concentration of alpha-amylase in a body fluid may determined by a kinetic assay employing a coupled enzymatic reaction system, such that a high degree of sensitivity and of uniformity in result may be afforded, while at the same time providing an assay that is relatively simple to carry out, that is rapid, and which does not require the employment of a specimen blank.

In accordance with this invention, these and other objects of the invention are obtained by performing the alpha-amylase assay by reacting a maltodextrin phosphorylase limit dextrin with alpha-amylase in the presence of maltodextrin phosphorylase, with resultant formation of fragments of limit dextrin which concomitantly react with the maltodextrin phosphorylase to form the initial component of a coupled enzymatic reaction system that is specific for said component, and which results in the formation of a chromophore, simultaneously performing the reactions of said coupled enzymatic reaction system responsive to the formation of said component, and measuring the rate of formation of said chromophore, said alpha-amylase being rate limiting.

Starch consists in nature of a combination of two polymers, namely, alpha-amylose and amylopectin. Alpha-amylose consists essentially of long unbranched chains in which all of the D-glucose units are bound by alpha-1,4 linkages, namely, linkages which contain an oxygen atom and which link the first carbon atom of a glucose unit with the fourth carbon atom of an immediately adjacent glucose unit. The

chains are polydisperse and vary in molecular weight from a few thousand to around 50 000, there being a free, namely unlinked, non-reducing terminal at one end and a free reducing terminal at the other end.

Amylopectin, unlike amylose, is highly branched. In a typical amylopectin, the branches or chains contain, on the average, twelve glucose units successively connected by alpha-1,4 linkages, so that branch points occur on the average of every twelfth glucose unit. At the branch points the chains of alpha-1,4 linked glucose units are linked respectively to the first, fourth and sixth atoms of a single glucose unit, the occurrence of the 1-6 linkage with the sixth carbon atom being the characterizing structural feature at the branch points. The molecular structure of amylopectin normally comprises at its periphery a multiplicity of branches that are linked at one end to branch points and that have a free non-reducing terminal at the remote end with the exception of a single branch that has a free reducing terminal at the remote end. Amylopectin is further characterized by a polysaccharide core comprised of multiplicity of chains and branches made up of alpha-1,4 linked glucose units, which chains interconnect and are terminally linked at each end to branch points and, therefore, do not have a free non-reducing terminal of a free reducing terminal.

When amylose is hydrolyzed by the action thereon of alpha-amylase, the resulting cleavage of the alpha-1,4 linkages may occur anywhere along the amylose chain in such a way as to ultimately yield a mixture of oligosaccharides and maltose. Because of its ability to cleave interior linkages as well as linkages occurring near free non-reducing terminals alpha-amylase is referred to as an endocarbohydrase. Amylose may also be attacked by enzymes known as exo-carbohydrases, such as beta-amylase, glucoamylase and various phosphorylases. These enzymes cleave away successive carbohydrate monomers beginning only at a free non-reducing terminal of a chain of successive alpha-1,4 linked glucose units.

Because of its branched molecular structure, amylopectin is attacked in different ways by alpha-amylase and by exo-carbohydrases. Exo-carbohydrases cannot hydrolyze the alpha-1,6 linkages that occur at the branch points. Alpha-amylase, being an endo-carbohydrase, has the capacity to cleave alpha-1,4 linkages not only near free non-reducing chain terminals but also randomly at alpha-1,4 linkages between the chain terminals in the interior core portion of amylopectin. Exo-carbohydrases, on the other hand, can only attack alpha-1,4 linked glucopolysaccharides that contain free non-reducing terminals so as to cleave off a carbohydrate monomer followed by successively cleaving off additional monomers while progressing toward the alpha-1,6 branch point of an attacked branch. Eventually the enzymatic activity of an exo-carbohydrase on starch will cease.

The molecularly large carbohydrate product that results from the exhaustive reaction of amylopectin with an exo-carbohydrase is known in the art as a limit dextrin (Biochem. J., *76*, 264, 1960). In general, the structure of the limit dextrin may be considered as being an amylopectin core comprising alpha-1,6 branch sites interconnected by chains of alpha-1,4 glucose that are terminally linked to the alpha-1,6 branch sites. However, the ability of various exo-carbohydrases to attack the chains having free non-reducing terminals varies ; as a consequence, depending upon the exocarbohydrase employed, the amylopectin core will have attached to it short residual alpha-1,4 glucose chains of differing lengths. That is, the limit dextrin obtained with one exo-carbohydrase, e. g., beta-amylase, is not identical to a limit dextrin obtained with another exo-carbohydrase, e. g., maltodextrin phosphorylase. For example, beta-amylase is less effective than maltodextrin phosphorylase in attacking the alpha-1,4 linked glucose chains, accordingly, the limit dextrin obtained with beta-amylase has longer residual chains than the limit dextrin obtained with maltodextrin phosphorylase. As one consequence of this differing enzyme activity, the limit dextrin obtained with beta-amylase, although no longer capable of acting as a substrate for beta-amylase, is capable of acting as a substrate for maltodextrin phosphorylase. On the other hand, the limit dextrin obtained with maltodextrin phosphorylase cannot act as a substrate for either beta-amylase or maltodextrin phosphorylase. The limit dextrin which is employed in accordance with this invention is one which will not serve as a substrate for maltodextrin phosphorylase, and will be referred to herein as « maltodextrin phosphorylase limit dextrin », ore more simply as « MDP limit dextrin ».

The MDP limit dextrin employed in accordance with this invention need not necessarily be prepared from starch ; for example, it may be produced from glycogen, which is a storage polysaccharide found in animal tissues, being especially abundant in liver and muscle. Glycogen is similar to amylopectin in that it is a polysaccharide characterized by chains of alpha-1,4 linked glucose units. However, it is more highly branched with alpha-1,6 branch points occurring at intervals of about 8 to 10 glucose units. As in the case of amylopectin, glycogen contains chains of alpha-1,4 linked glucose units which chains have their terminal extremities linked to the branch points. Accordingly, when glycogen is exhaustively treated with an exo-carbohydrase, the resulting product is a limit dextrin essentially similar to that produced from starch with that exo-carbohydase.

In accordance with this invention, MDP limit dextrin is employed at the substrate for alpha-amylase comprised in a specimen to be analyzed, such as a sample of serum or urine, thereby initiating a series of reactions resulting in a chromophore response, the amount of the alpha-amylase being rate limiting and the rate of chromophore response being a measure of the concentration of the alpha-amylase in the specimen.

It is a further feature of this invention that the attack of the alpha-amylase on the MDP limit dextrin substrate therefor occurs in the presence of maltodextrin phosphorylase. It is inherent from the molecular

structure of MDP limit dextrin, that the attack on it by alpha-amylase in the specimen is limited essentially to ruptures of the chains of alpha-1,4 linked glucose units that have their terminal extremities linked to the alpha-1,6 branch points therein, with formation of polysaccharide or oligosaccharide fragments of MDP limit dextrin having newly formed free non-reducing chain terminals which are available for reaction with the maltodextrin phosphorylase. The maltodextrin phosphorylase is provided in excess and in sufficient amount to keep the alpha-amylase in the specimen being analyzed as the rate limiting constituent, whereby, as each new free non-reducing terminal is formed, it is attacked by the maltodextrin phosphorylase. Since the rate at which the non-reducing terminals are newly formed is responsive to the rate-limiting concentration of the alpha-amylase in the specimen, it follows that the rate of formation of the reaction products resulting from the attack of the maltodextrin phosphorylase on the non-reducing terminals as they are formed likewise is responsive to the rate limiting concentration of the alpha-amylase specimen.

As is well known, maltodextrin phosphorylase can be derived from mutant E. coli. See, e. g., Buehner, « Crystallization and Crystallographic Data of Escherichia coli Maltodextrin Phosphorylase », FEBS Letters, Vol. 85, No. 1, 914 (1978) and Thanner et al, « Hydrophobic and Biospecific Chromatography in the Purification of Maltodextrin Phosphorylase from E. coli », FEBS Letters, Vol. 55, No. 1, 178 (1975). However, maltodextrin phosphorylase obtained from other sources may be used in the practice of this invention.

Maltodextrin phosphorylase is used in the presence of inorganic phosphate, and it reacts with alpha-amylase fragmented MDP limit dextrin to form glucose-1-phosphate from the newly exposed non-reducing ends as and to the extent that the limit dextrin is attacked by alpha-amylase comprised in the specimen. The rate of formation of glucose-1-phosphate may be measured by any suitable coupled enzymatic reaction system. The preferred enzymatic reaction system that is employed is as follows :

$$\text{MDP limit dextrin} \xrightarrow{\text{alpha-amylase}} \text{fragmented MDP limit dextrin.} \qquad (1)$$

$$\text{Inorganic phosphate + fragmented MDP limit dextrin} \xrightarrow{\text{MDP}} \text{glucose-1-phosphate + residual MDP limit dextrin fragments.} \qquad (2)$$

$$\text{Glucose-1-phosphate} \xrightarrow{\text{phosphoglucomutase}} \text{glucose-6-phosphate.} \qquad (3)$$

$$\text{NAD + glucose-6-phosphate} \xrightarrow[\text{dehydrogenase}]{\text{glucose-6-phosphate}} \text{NADH + 6-phosphogluconate.} \qquad (4)$$

The inorganic phosphate used as a source of phosphate ions may be conveniently supplied by performing the reaction in an aqueous medium containing a phosphate buffer.

The amounts of the respective enzymatic reagents should be such that the alpha-amylase is the rate limiting constituent. NAD is the chromophore forming agent and the rate of its conversion to NADH provides a quantitative measure of the concentration of the alpha-amylase in the specimen. The rate of such conversion normally is measured by measuring the rate of change in absorbence of light of given wave length using an instrument such as a spectrophotometer.

It is a significant advantage of this invention that the enzymatic reactions occurring in the above-mentioned reaction system are essentially unaffected by the presence of the maltose, or other saccharides, such as glucose, which may be present in the specimen, or which may be present in the reagents. For example, carbohydrates such as maltose can be employed as stabilizers or fillers for lyophylized enzyme reagents without effect on the analytical procedure of this invention.

The reactions ordinarily are performed when the reaction system is at a temperature between 18 °C and 40 °C and the pH is between 5 and 8. So long as the alpha-amylase is the rate limiting component of the system the concentrations of the other components are not critical. The reactions occur simultaneously and are triggered by the introduction of the alpha-amylase containing specimen into the aqueous medium containing the other reaction components.

This is because the majority of the cleavage of the carbohydrate source is effected by beta-amylase, and maltose is the major « by-product » of this reaction. Since maltose does not affect the analysis, presence of maltose as an impurity is not a particular problem. On the other hand, the glucose-1-phosphate « by-product » formed by incubation of the carbohydrate source with maltodextrin phosphorylase, can materially interfere with the analysis. Thus, use of maltodextrin phosphorylase only as the final step in the production of MDP limit dextrin reduces the amount of glucose-1-phosphate which is formed, and minimizes the amount of purification of the MDP limit dextrin which is required before use in accordance with this invention.

The MDP limit dextrin as initially produced in an aqueous medium may be employed by adding it to the aqueous medium comprising the remaining components of the overall reaction system of the assay.

For convenience in handling, the MDP limit dextrin may be reduced to the solid state by lyophilizing. It may be lyophilized *per se* or in combination with one or more components of the reaction system that is employed. When employed in combination, the basic combination used in any of the reaction system is the MDP limit dextrin with the maltodextrin phosphorylase. To the basic combination there may be added any one or more of the rest of the components of the selected coupled enzymatic reaction system. Preferably, all the rest of the reaction components are present in the lyophilized state so that at the time the assay is performed all that is required is the addition of water to form the aqueous medium of desired concentration and pH to which the alpha-amylase specimen may be added when the aqueous medium is at the desired temperature. However, if desired, the various components, either individually or in subcombinations, may be provided as contained in a kit for admixture at the time the assay is carried out, to the end that the involved coupled reactions may occur simultaneously so as to form the chromophore at a rate which is limited by the alpha-amylase that is added to complete the reagent system and thereby trigger the involved reactions.

The following example is illustrative of preferred practice of the invention. The following combination is initially prepared :

| | |
|---|---|
| Starch solution | 50 g/liter |
| Phosphate buffer | 50 mM |
| Beta-amylase | 7 200 U |
| pH | 6.9 |

The foregoing composition is incubated for 18 hours at 37 °C. 2 100 U of maltodextrin phosphorylase then is added and incubation is continued at 37 °C for 24 hours. Thereafter, heat to boiling to destroy the beta-amylase and the phosphorylase, and then dialyze to remove glucose-1-phosphate.

Using MDP limit dextrin prepared as above exemplified, the following assay composition is provided :

A. Reaction composition

| Component | Preferred Range | Optimum Combination |
|---|---|---|
| MDP limit dextrin | 0.1—20 mg/ml | 3 mg/ml |
| maltodextrin phosphorylase | 1—10 U/ml | 3 U/ml |
| phosphoglucomutase | 0.1—10 U/ml | 2 U/ml |
| glucose-6-phosphate dehydrogenase | 0.5—10 U/ml | 2 U/ml |
| NAD | 0.1—10 mM | 5 mM |
| phosphate buffer | 10—500 mM | 10 mM |
| glucose-1,6-diphosphate | 0.001—1.0 mM | 0.01 mM |
| magnesium chloride | 0.2—50 mM | 50 mM |
| ethylene diamine tetraacetic acid | 0.1—50 mM | 5 mM |
| pH | 5—8 | 6.9 |

B. Reaction conditions

1.0 ml of the reaction composition is incubated to 30 °C. Add 0.01 ml of alpha-amylase specimen in the form of plasma, serum or urine, mix and record the change in absorbence at 340 mm for 10 minutes using an instrument such as a spectrophotometer. Prepare a curve of change in absorbence per unit time as defined by readings during this period and based on the linear portion of the curve calculate alpha-amylase activity follows :

$$\text{U/l alpha-amylase} = \frac{\Delta A\ 340/\text{min} \times 1\,000 \times V_t \times 1\,000}{6\,220 \times \text{L.P.} \times V_s}$$

when

$\Delta$ A 340 = change in absorbence at 340 nm

min = minute

$V_t$ = total volume = 1.01 ml

$V_s$ = sample volume = 0.01 ml

L.P. = light path length = 1.0 cm

U/l alpha-amylase = 16.238 × $\Delta$A 340/min

Each unit of alpha-amylase activity is defined as that amount of enzyme which catalyzes the production of 1 μ mole of NADH per minute at 30 °C. The described assay is linear to 1 000 U/l.

In carrying out the foregoing example the specimen volume may be varied, e. g., from 0.001 to 0.1 ml per ml of reagent, depending on the sensitivity required and the capability of the instrument used to measure the rate.

In the foregoing formulation, magnesium chloride is a preferred ingredient which is included in order to promote the activity of the maltodextrin phosphorylase by modifying the activity of the phosphoglucomutase so as to promote the enzymatic activity of the phosphorylase. Other cations such as manganese, cobalt, zinc or nickel may be employed for similar purpose. The ethylene diamine tetraacetic acid is preferably included in the composition because of its influence in controlling relative concentrations of divalent cations required for the coupled enzymatic reactions. Glucose-1,6-diphosphate is included in the composition as a co-factor influencing the conversion of glucose-1-phosphate to glucose-6-phosphate in the presence of phosphoglucomutase. Since it also appears to influence the activity of maltodextrin phosphorylase, it should be present in an amount sufficient to activate both enzymes. The composition of the preferred example may also be provided in the lyophilized state wherein the components are present in like relative proportions adapted to provide the concentrations as above given upon reconstitution with water and preferably is supplied in this way for use in clinical testing laboratories that use either manual or mechanized assay techniques. However, the individual components singly or in various subcombinations may be supplied for like purpose and in like relative amounts in the form of a kit.

**Claims**

1. A reagent composition to be used in an alpha-amylase assay comprising :

(a) limit dextrin that is a substrate for said alpha-amylase and that is fragmentable by said alpha-amylase to form limit dextrin fragments having chains with non-reducing terminals ; and

(b) an exocarbohydrase in effective amount to react with the chains having said non-reducing terminals ;

and characterized in that :

(1) said limit dextrin is maltodextrin phosphorylase (MDP) limit dextrin ;

(2) said exocarbohydrase is maltodextrin phosphorylase effective to react with said fragments to form glucose-1-phosphate ; and

(3) said composition includes inorganic phosphate.

2. A reagent system to be used in an alpha-amylase assay comprising

(a) a reagent composition as defined in Claim 1 ; and

(b) a coupled enzymatic reaction system for forming a chromophore, which enzymatic reaction system is specific for and responsive to glucose-1-phosphate that is formed by reaction between said phosphorylase and said limit dextrin fragments in the presence of said inorganic phosphate ;

the amounts of said limit dextrin, said phosphorylase, and said phosphate forming said reagent composition, and the amount of said coupled enzymatic system being such that, when said reagent system is admixed with a specimen containing alpha-amylase, said limit dextrin is fragmented by alpha-amylase to form limit dextrin fragments which react with said phosphorylase to form glucose-1-phosphate, and whereby the amount of alpha-amylase contained in the specimen to be assayed is rate limiting.

3. A reagent system according to Claim 2 wherein said enzymatic reaction system comprises phosphoglucomutase, glucose-6-phosphate dehydrogenase, phosphate buffer and NAD.

4. A reagent system according to Claim 3 which also comprises ethylene diamine tetraacetic acid, glucose-1,6-diphosphate, and a cation selected from the group consisting of magnesium, manganese, zinc, nickel and cobalt.

5. A lyophilized reagent system for an alpha-amylase assay according to the reagent system set forth in Claim 2.

6. A reagent system according to Claim 4 comprising an aqueous composition, the composition having

| | |
|---|---|
| MDP limit dextrin | 0.1-20 mg/ml |
| maltodextrin phosphorylase | 1-10 U/ml |
| phosphoglucomutase | 0.1-10 U/ml |
| glucose-6-phosphate dehydrogenase | 0.5-10 U/ml |
| NAD | 0.1-10 mM |
| phosphate buffer | 10-500 mM |
| glucose-1,6-diphosphate | 0.001-0.1 mM |
| magnesium chloride | 0.2-100 mM |
| ethylene diamine tetraacetic acid | 0.1-50 mM |
| pH | 5-8 |

7. A non-aqueous reagent composition which when dissolved in water will produce the reagent system as set forth in Claim 6.

8. A lyophilized composition reconstitutable with water to provide the reagent system defined in Claim 6.

9. A reagent system according to Claim 2, wherein the limit dextrin is produced by exhaustively treating a carbohydrate selected from the group consisting of starch and glycogen and mixtures thereof in an aqueous medium with beta-amylase, followed by further treating the so-treated carbohydrate with maltodextrin phosphorylase in the presence of inorganic phosphate, and destroying said beta-amylase and said phosphorylase after said treatment therewith by heating said aqueous medium.

10. A reagent system according to Claim 6 wherein the limit dextrin is produced by exhaustively treating a carbohydrate selected from the group consisting of starch and glycogen and mixtures thereof in an aqueous medium with beta-amylase, followed by further treating the so-treated carbohydrate with maltodextrin phosphorylase in the presence of inorganic phosphate, and destroying said beta-amylase and said phosphorylase after said treatment therewith by heating said aqueous medium.

11. A lyophilized composition reconstitutable with water to provide the reagent system defined in Claim 10.

12. An alpha-amylase assay which comprises

(a) reacting an alpha-amylase specimen with a reagent composition as defined in Claim 1 to form limit dextrin fragments which concomitantly react with said maltodextrin phosphorylase to form glucose-1-phosphate as the initial component of a coupled enzymatic reacting system that is specific thereto and that results in the formation of a chromophore ;

(b) simultaneously performing the reactions of said coupled enzymatic reaction system responsive to the formation of said component ; and

(c) measuring the rate of formation of said chromophore ;

the proportions of the limit dextrin, phosphorylase and phosphate forming said reagent composition to said alpha-amylase being such that the amount of said alpha-amylase is rate limiting.

13. An alpha-amylase assay according to Claim 12 wherein the performance of said coupled enzymatic reaction system comprises converting said glucose-1-phosphate to glucose-6-phosphate in the presence of phosphoglucomutase, and converting said glucose-6-phosphate to 6-phosphogluconate in the presence of glucose-6-phosphate dehydrogenase and the coenzyme NAD with concomitant conversion of said NAD to NADH.

14. An alpha-amylase assay according to Claim 13 wherein the reagent system also contains ethylene diamine tetraacetic acid, glucose-1,6-diphosphate and a cation selected from the group consisting of magnesium, manganese, zinc, nickel and cobalt.

**Patentansprüche**

1. Reagenz zur Bestimmung von alpha-Amylase enthaltend :

(a) Grenzdextrin als Substrat für die alpha-Amylase, das durch die alpha-Amylase zu Grenzdextrinfragmenten mit nicht reduzierenden Kettenenden aufgespalten werden kann und

(b) eine Exocarbohydrase in wirksamer Menge um mit den nicht reduzierenden Kettenenden zu reagieren

dadurch gekennzeichnet, daß

(1) das Grenzdextrin ein Maltodextrin-Phosphorylase (MDP) Grenzdextrin ist,

(2) die Exocarbohydrase Maltodextrin-Phosphorylase ist, die geeignet ist zur Umsetzung mit den Fragmenten unter Bildung von Glucose-1-phosphat und

(3) das Reagenz anorganisches Phosphat enthält.

2. Reagenzsystem zur Bestimmung von alpha-Amylase enthaltend

(a) ein Reagenz gemäß Anspruch 1 und

(b) ein gekoppeltes Enzym-Reaktionssystem zur Bildung eines Chromophors, das spezifisch ist für durch Umsetzung von Phosphorylase und Grenzdextrinfragmenten in Gegenwart des Anorganischen Phosphates gewonnenen Glucose-1-phosphat und das auf das Glucose-1-phosphat reagiert,

wobei die Menge des Grenzdextrins, der Phosphorylase und des Phosphats im Reagenz und die Menge des gekoppelten Enzymsystems derart bemessen ist, daß beim Vermischen des Reagenzsystems mit der Alpha-Amylase enthaltenden Probe, das Grenzdextrin durch die alpha-Amylase zu Grenzdextrinfragmenten aufgespalten wird, die mit der Phosphorylase unter Bildung von Glucose-1-phosphat reagieren, wobei die Menge der alpha-Amylase in der zu untersuchenden Probe die Geschwindigkeit begrenzt.

3. Reagenzsystem nach Anspruch 2, dadurch gekennzeichnet, daß das Enzym-Reaktionssystem Phosphorglucomutase, Glucose-6-phosphat-Dehydrogenase, Phosphatpuffer und NAD enthält.

4. Reagenzsystem nach Anspruch 3, dadurch gekennzeichnet, daß es zusätzlich Äthylendiamintetraessigsäure, Glucose-1,6-diphosphat und Magnesium-, Mangan-, Zink-, Nickel- oder Kobaltkationen enthält.

5. Lyophilisiertes Reagenzsystem zur Bestimmung von alpha-Amylase gemäß dem Reagenzsystem nach Anspruch 2.

6. Reagenzsystem nach Anspruch 4, enthaltend ein wäßriges Gemisch der folgenden Zusammensetzung :

| | |
|---|---|
| MDP-Grenzdextrin | 0,1 bis 20 mg/ml |
| Maltodextrin-Phosphorylase | 1 bis 10 U/ml |
| Phosphoglucomutase | 0,1 bis 10 U/ml |
| Glucose-6-phosphat-Dehydrogenase | 0,5 bis 10 U/ml |
| NAD | 0,1 bis 10 mM |
| Phosphatpuffer | 10 bis 500 mM |
| Glucose-1,6-diphosphat | 0,001 bis 0,1 mM |
| Magnesiumchlorid | 0,2 bis 100 mM |
| Äthylendiamintetraessigsäure | 0,1 bis 50 mM |
| pH | 5 bis 8 |

7. Nicht-wäßriges Reagenz, dadurch gekennzeichnet, daß es beim Auflösen in Wasser das Reagenzsystem gemäß Anspruch 6 bildet.

8. Lyophilisiertes Gemisch, dadurch gekennzeichnet, daß sich daraus mit Wasser das Reagenzsystem gemäß Anspruch 6 herstellen läßt.

9. Reagenzsystem nach Anspruch 2 zur Herstellung von Grenzdextrin, dadurch gekennzeichnet, daß man Stärke oder Glykogen oder ein Gemisch davon in einem wäßrigen Medium mit beta-Amylase erschöpfend behandelt, darauf das so behandelte Carbohydrat mit Maltodextrin-phosphorylase in Gegenwart von anorganischem Phosphat weiter behandelt und die beta-Amylase und Phosphorylase nach dieser Behandlung durch Erhitzen des Wäßrigen Mediums zerstört.

10. Reagenzsystem nach Anspruch 6 zur Herstellung von Grenzdextrin, dadurch gekennzeichnet, daß man Stärke oder Glykogen oder ein Gemisch davon in einem Wäßrigen Medium mit β-Amylase erschöpfend behandelt, darauf das so behandelte Carbohydrat mit Maltodextrinphosphorylase in Gegenwart von anorganischem Phosphat weiterbehandelt und die beta-Amylase und Phosphorylase nach dieser Behandlung durch Erhitzen des wäßrigen Mediums zerstört.

11. Lyophilisiertes Gemisch, dadurch gekennzeichnet, daß sich daraus mit Wasser das Reagenzsystem gemäß 10 herstellen läßt.

12. Verfahren zur Bestimmung von alpha-Amylase, dadurch gekennzeichnet, daß man

(a) eine alpha-Amylase-Probe mit einem Reagenz gemäß Anspruch 1 unter Bildung von Grenzdextrinfragmenten umsetzt, welche gleichzeitig mit der Maltodextrin-phosphorylase unter Bildung von Glucose-1-phosphat als Indikatorkomponente eines gekoppelten Enzym-Reaktionssystems reagiert, welches für die Indikatorkomponente spezifisch ist und einen Chromophor bildet,

(b) gleichzeitig die Reaktionen des gekoppelten Enzym-Reaktionssystems durchführt, das auf die Bildung der Indikatorkomponente reagiert und

(c) die Geschwindigkeit der Bildung des Chromophors mißt

wobei die Mengenverhältnisse des Grenzdextrins, der Phosphorylase und des Phosphats in Reagenz zur alpha-Amylase derart sind, daß die Menge der alpha-Amylase die Geschwindigkeit begrenzt.

13. Verfahren zur Bestimmung von alpha-Amylase nach Anspruch 12, dadurch gekennzeichnet, daß in dem gekoppelten Enzym-Reaktionssystem das Glucose-1-phosphat zu Glucose-6-phosphat in Gegenwart von Phosphoglucomutase und das Glucose-6-phosphat in Gegenwart von Glucose-6-phosphatdehydrogenase und NAD als Coenzym zu 6-Phosphogluconat bei gleichzeitiger Umsetzung von NAD zu NADH umgesetzt wird.

14. Verfahren zur Bestimmung von alpha-Amylase nach Anspruch 13, dadurch gekennzeichnet, daß das Reaktionssystem auch Äthylendiamintetraessigsäure, Glucose-1,6-diphosphat und Magnesium-, Mangan-, Zink-, Nickel- oder Kobaltkationen enthält.

**Revendications**

1. Composition réactive destinée à être utilisée dans un dosage d'alpha-amylase comprenant :

(a) une dextrine limite qui est un substrat pour ladite alpha-amylase et qui peut être fragmenté par action de ladite alpha-amylase pour former des fragments de dextrine limite ayant des chaînes avec des terminaux non réducteurs ; et

(b) une exo-carbohydrase en quantité efficace pour réagir avec les chaînes ayant des terminaux non réducteurs ;

et caractérisée en ce que :

(1) ladite dextrine limite est la dextrine limite de phosphorylase de maltodextrine (MDP) ;

(2) ladite exo-carbohydrase est une phosphorylase de maltodextrine efficace pour réagir avec lesdits fragments pour former du glucose-1-phosphate ; et

(3) ladite composition comprend du phosphate minéral.

2. Système réactif destiné à être utilisé dans un dosage d'alpha-amylase comprenant :

(a) une composition de réactifs définie selon la revendication 1 et

(b) un système réactionnel enzymatique couplé pour former un chromophore, lequel système

réactionnel enzymatique est spécifique et sensible au glucose-1-phosphate formé par réaction entre ladite phosphorylase et lesdits fragments de dextrine limite, en présence dudit phosphate minéral ; les quantités de ladite dextrine limite, de ladite phosphorylase et dudit phosphate formant ladite composition réactive et la quantité dudit système enzymatique couplé étant telle que lorsque ledit système de réactifs est mélangé avec un échantillon contenant de l'alpha-amylase, ladite dextrine limite est fragmentée par l'alpha-amylase pour former des fragments de dextrine limite qui réagissent avec ladite phosphorylase pour former du glucose-1-phosphate d'où il résulte que la quantité d'alpha-amylase contenue dans l'échantillon à doser limite le taux.

3. Système réactif selon la revendication 2, caractérisé en ce que ledit système réactionnel enzymatique comprend le phosphoglucomutase, le glucose-6-phosphate déhydrogénase, un tampon phosphate et le NAD.

4. Système réactif selon la revendication 3, qui comprend également de l'acide éthylènediamine tétraacétique du glucose-1,6-diphosphate et un cation choisi dans le groupe constitué par le magnésium, le manganèse, le zinc, le nickel et le cobalt.

5. Système réactif lyophilisé pour le dosage d'une alpha-amylase selon le système réactif de la revendication 2.

6. Système réactif selon la revendication 4, comprenant une composition aqueuse, la composition comportant :

| | |
|---|---|
| 0,1 à 20 mg/ml | de dextrine limite MDP |
| 1 à 10 U/ml | de phosphorylase de maltodextrine |
| 0,1 à 10 U/ml | de phosphoglucomutase |
| 0,5 à 10 U/ml | de glucose-6-phosphate déhydrogénase |
| 0,1 à 10 mM | de NAD |
| 10 à 500 mM | de tampon au phosphate |
| 0,001 à 0,1 mM | de glucose-1,6-diphosphate |
| 0,2 à 100 mM | de chlorure de magnésium |
| 0,1 à 50 mM | d'acide éthylènediamine tétraacétique |
| pH de 5 à 8 | |

7. Composition réactive non aqueuse qui, lorsqu'elle est dissoute dans l'eau produit le système réactif selon la revendication 6.

8. Composition lyophylisée reconstituable avec de l'eau pour donner le système réactif selon la revendication 6.

9. Système réactif selon la revendication 2, dans lequel la dextrine limite est produite par traitement exhaustif d'un carbohydrate choisi dans le groupe constitué par l'amidon et le glycogène et leurs mélanges en milieu aqueux avec la bêta-amylase, suivi par un traitement ultérieur du carbohydrate ainsi traité avec de la phosphorylase de maltodextrine en présence de phosphate minéral et destruction de ladite bêta-amylase et de ladite phosphorylase après traitement en chauffant ledit milieu aqueux.

10. Système réactif selon la revendication 6, dans lequel la dextrine limite est produite par traitement exhaustif d'un carbohydrate choisi dans le groupe constitué par l'amidon et le glycogène et leurs mélanges en milieu aqueux avec de la bêta-amylase suivi d'un traitement ultérieur du carbohydrate ainsi traité avec de la phosphorylase de maltodextrine en présence de phosphate minéral et destruction de ladite bêta-amylase et de ladite phosphorylase après le susdit traitement en chauffant ledit milieu aqueux.

11. Composition lyophilisée reconstituable avec de l'eau pour donner le système réactif selon la revendication 10.

12. Dosage d'alpha-amylase qui comprend :

(a) la réaction d'un échantillon d'alpha-amylase avec une composition réactive définie selon la revendication 1, pour former des fragments de dextrine limite qui réagissent de façon concomitante avec ladite phosphorylase de maltodextrine pour former le glucose-1-phosphate comme composant initial d'un système réactionnel enzymatique couplé qui est spécifique et qui résulte dans la formation d'un chromophore ;

(b) la mise en œuvre simultanée des réactions dudit système réactionnel enzymatique couplé sensible à la formation dudit composant ; et

(c) la mesure du taux de formation dudit chromophore ;

les proportions de la dextrine limite, de la phosphorylase, du phosphate formant ladite composition réactive vis-à-vis de l'alpha-amylase étant telles que la quantité de ladite alpha-amylase limite le taux.

13. Dosage d'alpha-amylase selon la revendication 12, dans lequel la mise en œuvre du système réactionnel enzymatique couplé comprend la conversion dudit glucose-1-phosphate au glucose-6-phosphate en présence de phosphoglucomutase et la conversion dudit glucose-6-phosphate en 6-phosphogluconate, en présence de glucose-6-phosphate déhydrogénase et le co-enzyme NAD avec la conversion concomitante dudit NAD en NADH.

14. Dosage d'alpha-amylase selon la revendication 13, dans lequel le système réactif contient également de l'acide éthylènediamine tétra-acétique, du glucose-1,6-diphosphate et un cation choisi dans le groupe constitué par le magnésium, le manganèse, le zinc, le nickel et le cobalt.